# EUROPÄISCHE PATENTSCHRIFT

(11) **EP 2 382 460 B1**
(45) Veröffentlichungstag und Bekanntmachung des Hinweises auf die Patenterteilung: **28.12.2016**
(21) Anmeldenummer: 10701505.9
(22) Anmeldetag: 22.01.2010
(51) Int. Cl.: G01N 33/00, G01N 27/70

(54) **VERFAHREN UND VORRICHTUNG ZUR DETEKTION VON IONISIERBAREN GASEN**
METHOD AND DEVICE FOR DETECTING GASES THAT CAN BE IONIZED
PROCÉDÉ ET DISPOSITIF DE DÉTECTION DE GAZ IONISABLES

(30) Priorität: 23.01.2009 DE 102009006016
(43) Veröffentlichungstag der Anmeldung: 02.11.2011
(73) Patentinhaber: PlasmaTreat GmbH, 33803 Steinhagen (DE)
(72) Erfinder: BUSKE, Christian, 33619 Bielefeld (DE)
(74) Vertreter: Cohausz & Florack
(86) Internationale Anmeldenummer: PCT/EP2010/050713
(87) Internationale Veröffentlichungsnummer: WO 2010/084166

(56) Entgegenhaltungen:
- EP-A1- 1 279 955
- EP-A1- 1 557 667
- DE-A1-102005 018 926

## Beschreibung

Die Erfindung betrifft ein Verfahren zur Detektion von ionisierbaren Gasen, insbesondere organischen Molekülen, vorzugsweise Kohlenwasserstoffen. Die Erfindung betrifft auch eine Abgasreinigungsanlage mit einer Vorrichtung zur Detektion von ionisierbaren Gasen mit einem Gaseinlass, mit Mitteln zur Ionisation eines ionisierbaren Gases, mit einer Spannungsquelle, mit zwei Elektroden und mit Mitteln zur Bestimmung einer Stromstärke, wobei die zwei Elektroden an die Spannungsquelle angeschlossen sind und wobei die Mittel zur Bestimmung einer Stromstärke so an die Elektroden angeschlossen sind, dass die Stärke des zwischen den Elektroden fließenden Stroms messbar ist.

Zur Detektion organischer Moleküle, beispielsweise von Kohlenwasserstoffen in einem Gasgemisch, wird häufig das Verfahren der Flammenionisationsdetektion eingesetzt. Bei einem solchen Verfahren wird das zu untersuchende Gasgemisch in eine Flamme, insbesondere in eine Knallgasflamme eingeleitet. Dort werden die ionisierbaren Bestandteile des Gasgemisches, also insbesondere organische Moleküle, durch die thermische Energie ionisiert. Die so entstehenden freien Elektronen und Ionen, beispielsweise CHO⁺, bewegen sich dann entsprechend ihrer Ladung jeweils auf eine von zwei vorgesehenen Elektroden, zwischen denen eine Spannung angelegt ist, zu. Der auf diese Weise zwischen den Elektroden fließende Strom ist ein Maß für den Anteil des ionisierbaren Gases, also der Konzentration der organischen Moleküle, am gesamten Gasgemisch.

Das Verfahren der Flammenionisationsdetektion hat den Nachteil, dass für die Erzeugung der Flamme die Bereitstellung von Wasserstoff und Sauerstoff erforderlich ist. Weiterhin wird die Umgebung des Brenners durch die Flamme stark aufgeheizt. Ein weiterer Nachteil besteht darin, dass ein Teil der organischen Moleküle durch die Flamme oxidiert wird. Diese tragen dann nicht zu dem zwischen den Elektroden fließenden Strom bei und werden somit auch nicht detektiert. Das Verfahren ist somit ungenau.

Die Bestimmung der Konzentration organischer Moleküle in einem Gasgemisch ist unter anderem bei Abgasen erforderlich. So sind bei Abgasanlagen beispielsweise gesetzlich bestimmte Maximalkonzentrationen bestimmter organischer Stoffe einzuhalten. Zur Einhaltung derartiger Normen werden im Stand der Technik häufig geregelte Abgasreinigungsanlagen eingesetzt. Die für die Regelung erforderliche Messgröße der Konzentration der organischen Moleküle im Abgas wird dabei zum Beispiel mit Hilfe eines Flammenionisationsdetektors bestimmt. Diese haben jedoch den Nachteil, dass zum Betrieb dieser Detektoren ein brennbares Gas, beispielsweise Knallgas erforderlich ist. Je nach Position des Detektors in der Abgasanlage ist es aber aufwändig, gefährlich bzw. sogar unmöglich eine Versorgung mit dem brennbaren Gas zu gewährleisten.

Die Bestimmung der Konzentration organischer Moleküle in einem Gasgemisch ist auch bei allgemeiner Gasanalytik erforderlich, beispielsweise bei der Analyse eines Prozessgases. Der Nachteil bei der Verwendung von Flammenionisationsdetektoren liegt hier darin, dass das Prozessgas durch das nicht vollständig verbrannte brennbare Gas zum Betrieb der Flamme oder durch Verbrennungsprodukte verunreinigt wird. Dies kann negative Auswirkungen auf die der Analyse nachgelagerten Prozessschritte haben.

Aus der EP 1 279 955 A1 ist ein Heliumionisationsdetektor bekannt.

Der vorliegenden Erfindung liegt somit die technische Aufgabe zu Grunde, ein Verfahren und eine Vorrichtung zur Detektion von ionisierbaren Gasen, insbesondere organischen Molekülen, vorzugsweise Kohlenwasserstoffen zur Verfügung zu stellen, die die oben genannten Nachteile zumindest teilweise vermeidet.

Diese Aufgabe wird erfindungsgemäß mit einem Verfahren nach Patentanspruch 1 gelöst, bei dem ein atmosphärischer Plasmastrahl erzeugt wird, bei dem ein das ionisierbare Gas enthaltendes Gasgemisch mit dem Plasmastrahl in Wechselwirkung gebracht wird und bei dem ein elektrischer Strom als Maß für die Konzentration des ionisierbaren Gases in dem Gasgemisch gemessen wird.

Dadurch, dass das Gasgemisch mit einem atmosphärischen Plasmastrahl in Wechselwirkung gebracht wird, wird das darin enthaltene ionisierbare Gas durch den Plasmastrahl zumindest teilweise ionisiert. Die dadurch entstehenden freien Elektronen und Ionen stellen bewegliche Ladungsträger dar, durch die ein elektrischer Strom bewirkt wird, der als Maß für die Konzentration des ionisierbaren Gases in dem Gasgemisch gemessen wird. Die Ionisation des ionisierbaren Gases mit dem atmosphärischen Plasma hat gegenüber der thermischen Ionisation mit einer Flamme den Vorteil, dass kein brennbares Gas, beispielsweise Knallgas bzw. Wasserstoff und Sauerstoff zur Verfügung gestellt werden muss, um die Flamme zu erzeugen. So kann als Arbeitsgas zur Herstellung des atmosphärischen Plasmas Luft verwendet werden. Diese steht in der Regel als Umgebungsluft zur Verfügung. Weiterhin wird die Umgebung des Plasmastrahls durch den Plasmastrahl weniger aufgeheizt als die Umgebung einer Flamme, insbesondere einer Knallgasflamme durch die Flamme selbst.

In einer bevorzugten Ausführungsform des Verfahrens wird der elektrische Strom zwischen zwei Elektroden gemessen, wobei zwischen den Elektroden eine Spannung angelegt wird. Die Messung der elektrischen Größe wird auf diese Weise sehr einfach realisiert. So kann beispielsweise der zwischen den Elektroden fließende Strom, die Spannung oder die Kapazität der Elektrodenanordnung gemessen werden. Die Kapazität wird von den geladenen Teilchen beeinflusst, die sich zwischen den Elektroden befinden. Weiterhin können die Elektroden als Kapazität in einem Resonanzkreis integriert sein und dessen Resonanzfrequenz gemessen werden.

Eine weitere Ausführungsform des Verfahrens wird dadurch erreicht, dass zwischen den Elektroden eine Spannung zwischen 50 V und 350 V, insbesondere zwischen 50 V und 250 V, vorzugsweise zwischen 75 V und 150 V angelegt wird. Es hat sich gezeigt, dass das Verfahren besonders gut bei Spannungen in diesen Spannungsbereichen durchgeführt werden kann. Bei einer zu hohen Spannung besteht die Gefahr, dass zwischen den Elektroden ein zu hoher Strom fließt, so dass die verwendete Apparatur zur Bestimmung der Stromstärke beschädigt werden kann. Bei einer zu niedrigen Spannung ist der zwischen den Elektroden fließende elektrische Strom sehr klein, so dass die Messung der Stromstärke größere relative Fehler aufweist. Weiterhin können die Ionen und Elektronen bei einer zu geringen Spannung stark durch die bei der Ionisation entstehende Raumladungszone zwischen den Elektroden beeinflusst werden.

Bei dem Verfahren kann zwischen den Elektroden entweder eine Gleichspannung oder eine Wechselspannung angelegt werden. Bei einer Gleichspannung wandern die bei der Ionisation frei werdenden Elektronen und Ionen jeweils immer zu derselben Elektrode. Auf diese Weise ist eine kontinuierliche Messung des zwischen den Elektroden fließenden Stroms möglich. Das Anlegen einer Wechselspannung hat den Vorteil, dass das Signal der Stromstärkenmessung über die Wechselspannung moduliert werden kann. Dies erlaubt es, das Messsignal beispielsweise durch eine Lock-In-Verstärkung von Störeinflüssen zu trennen. Zwischen den Elektroden kann auch eine Rechteckspannung angelegt werden, um die Messungen zeitlich zu diskretisieren.

Eine rauschärmere Messung wird dadurch erreicht, dass der Plasmastrahl im Wesentlichen potentialfrei ist. Dadurch wird durch den Plasmastrahl kein zusätzliches elektrisches Feld in den Bereich der Ionisation eingebracht, durch das die freien Elektronen und Ionen beeinflusst werden.

Bei dem Verfahren wird der Plasmastrahl durch eine mit einer hochfrequenten Hochspannung erzeugten Bogenentladung hergestellt. Unter einer hochfrequenten Hochspannung wird dabei typischerweise eine Spannung im Bereich von 1 bis 50 kV, insbesondere 1 bis 15 kV bei einer Frequenz von 1 bis 100 kHz, insbesondere 10 bis 100 kHz, bevorzugt 10 bis 50 kHz verstanden. Ein derart hergestellter Plasmastrahl weist bei hoher Reaktivität eine besonders niedrige Temperatur auf, so dass die Umgebung des Plasmastrahls durch den Plasmastrahl nur geringfügig erwärmt wird.

Eine Beeinflussung des Gasgemisches durch das Detektionsverfahren wird dadurch reduziert, dass zur Erzeugung des Plasmastrahls ein inertes Arbeitsgas, vorzugsweise Stickstoff oder ein Edelgas, beispielsweise Argon oder Helium, verwendet wird. Auf diese Weise wird das Gasgemisch nicht durch nichtinerte Arbeitsgase verunreinigt. Dies ist insbesondere dann vorteilhaft, wenn das Verfahren zur Analytik von Gasströmen eingesetzt wird, wobei der Gasstrom nach der Analyse weiter verwendet wird, beispielsweise in einem nachfolgenden Prozessschritt. Dies bietet insbesondere auch Vorteile gegenüber den im Stand der Technik verwendeten Flammen, da der Gasstrom nicht durch nicht vollständig verbrannten Gase, beispielsweise Wasserstoff, oder durch die entstehenden Verbrennungsprodukte, beispielsweise Wasser oder Kohlendioxid, verunreinigt wird.

Erfindungsgemäß wird das Gasgemisch aus einem Abgasfluss abgeleitet. Bei einem Abgasfluss, beispielsweise aus einem Verbrennungsprozess, ist die Kontrolle der darin enthaltenen organischen Bestandteile wichtig, beispielsweise zur Einhaltung gesetzlicher Emissionsgrenzwerte. Daher ist es vorteilhaft, das Gasgemisch aus einem Abgasfluss mit dem Plasmastrahl in Wechselwirkung treten zu lassen, um auf diese Weise die Konzentration des ionisierbaren Gases in dem Abgasfluss zu bestimmen.

Eine weitere Form des Verfahrens ist dadurch gegeben, dass das Gasgemisch durch das Beaufschlagen einer verschmutzten Oberfläche mit dem Plasmastrahl erzeugt wird. Auf diese Weise erfüllt der Plasmastrahl gleichzeitig zwei Funktionen auf einmal. Durch den Plasmastrahl werden Verschmutzungen von der Oberfläche gelöst und dann als Gas in dem Plasmastrahl ionisiert. Der gemessene elektrische Strom stellt dann ein Maß für die von der Oberfläche entfernte Verschmutzung dar. Die Beaufschlagung der Oberfläche mit dem Plasmastrahl kann dann beispielsweise so lange durchgeführt werden, bis die Konzentration des ionisierbaren Gases unter einen gewissen Wert gesunken ist. Damit wird gewährleistet, dass bei der Reinigung der Oberfläche ein bestimmter Reinheitsgrad erreicht wird.

Bei einer leitenden, beispielsweise einer metallischen Oberfläche kann eine der Elektroden durch die Oberfläche ersetzt werden. In diesem Fall wird die Spannung zwischen der verbleibenden Elektrode und der Oberfläche angelegt. Alternativ kann auch auf die verbliebene Elektrode dadurch verzichtet werden, dass die Spannung zwischen der Oberfläche und der Plasmadüse angelegt wird.

Eine von der bisher beschriebenen Analyse des Gasgemisches, basierend auf einer elektrischen Größe, abweichende Analyse wird in einer weiteren Ausführungsform des Verfahrens dadurch erreicht, dass das im Wechselwirkungsbereich des Plasmastrahls mit dem Gasgemisch entstehende Licht analysiert wird und die Intensität des Lichtes in mindestens einem Spektralbereich als Maß für die Konzentration mindestens eines Stoffes im Gasgemisch bestimmt wird.

Im Bereich des Plasmas werden Stoffe des Gasgemisches, insbesondere einzelne Moleküle und Atome, durch die im Plasma enthaltene Energie angeregt und emittieren Licht. Da diese Stoffe ein charakteristisches wellenlängenabhängiges Emissionsverhalten aufweisen, lässt sich die Konzentration des Stoffes im Plasma anhand einer wellenlängenselektiven Analyse des vom Plasma abgestrahlten Lichtes bestimmen. Die Konzentration eines einzelnen Stoffes lässt sich dabei durch die Intensität des emittierten Lichtes in einem Spektralbereich ermitteln, in dem eine charakteristische Emissionswellenlänge des Stoffes liegt.

Bevorzugt wird zur Bestimmung der Spektren die Optische Emissionsspektroskopie (OES) eingesetzt, die eine weit verbreitete Technologie darstellt. Diese Spektroskopie besteht darin, einen Lichtstrahl mittels eines Beugungsgitters spektral zu zerlegen und anschließend mittels einer Zeilenkamera oder CCD-Kamera aufzuzeichnen. Die so ermittelten Spektren zeigen eine Intensitätsverteilung in Abhängigkeit von der Wellenlänge, so dass eine wellenlängenselektive Analyse des aus dem Plasma gewonnenen Lichtes ermöglicht wird. Selbstverständlich können auch andere Spektroskopen verwendet werden.

In bevorzugter Weise ist es weiterhin möglich, die Konzentration eines Stoffes gleichzeitig auf zwei unterschiedliche Weisen zu bestimmen, also sowohl anhand einer elektrischen Größe als auch anhand der spektralen Lichtausbeute. Dadurch kann eine Erhöhung der Genauigkeit erreicht werden. Alternativ kann das eine Messsignal zur Kalibrierung des anderen Signals eingesetzt werden. Weiterhin ist es möglich, durch die Analyse des Lichtes die Konzentration von Stoffen zu bestimmen, die im Plasmastrahl nicht oder nicht vollständig ionisiert werden.

Die Analyse des Lichtes ist besonders vorteilhaft bei der Beaufschlagung einer verschmutzten Oberfläche mit dem Plasmastrahl.

Die der Erfindung zu Grunde liegende Aufgabe wird weiterhin durch eine Abgasreinigungsanlage mit den Merkmalen des Patentanspruchs 7 gelöst.

Der Vorteil, als Mittel zur Ionisation von ionisierbaren Gasen eine Plasmadüse zur Erzeugung eines atmosphärischen Plasmastrahls vorzusehen, liegt darin begründet, dass zum Betrieb der Plasmadüse kein brennbares Gas, insbesondere Wasserstoff und/oder Sauerstoff bereitgehalten werden muss. Als Arbeitsgas zur Erzeugung des atmosphärischen Plasmastrahls kann Luft verwendet werden, welche beispielsweise aus der Umgebung der Vorrichtung entnommen werden kann. So ist ein Einsatz der Vorrichtung an Stellen möglich, an denen eine Versorgung mit einem brennbaren Gas oder die Versorgung mit einem anderen Arbeitsgas als Luft nicht möglich, aufwändig oder gefährlich ist. Der Gaseinlass kann so angeordnet sein, dass das Gasgemisch vor der Plasmadüse in den Plasmastrahl eingebracht wird. Es ist auch möglich, eine rohrartige Verlängerung des Auslassbereiches der Plasmadüse vorzusehen, in die das Gasgemisch eingeleitet wird.

Unter dem Gaseinlass wird also beispielsweise eine Zuleitung für das Gasgemisch verstanden. Alternativ kann die Vorrichtung jedoch auch so in einer Gasgemisch enthaltenden Umgebung, beispielsweise einem Gasfluss, angeordnet sein, dass das zu analysierende Gasgemisch durch Druck, Diffusion, Auftrieb oder auf ähnliche Weise zu der Vorrichtung gelangt. Der Gaseinlass wird dann durch mindestens eine offene, anströmbare Seite der Vorrichtung gebildet. Somit ist der Begriff des Gaseinlasses sehr breit zu verstehen.

In einer bevorzugten Ausführungsform der Vorrichtung ist mit der Spannungsquelle eine Spannung zwischen 50 V und 350 V, insbesondere zwischen 50 V und 250 V, vorzugsweise zwischen 75 V und 150 V erzeugbar. Es hat sich gezeigt, dass eine zwischen den Elektroden angelegte Spannung in diesen Spannungsbereichen für den Betrieb der Vorrichtung besonders gut geeignet ist.

Bei der Vorrichtung kann als Spannungsquelle eine Gleichspannungsquelle oder eine Wechselspannungsquelle vorgesehen sein. Mit einer Gleichspannungsquelle ist eine kontinuierliche Detektion der Konzentration des ionisierbaren Gases möglich, mit einer Wechselspannungsquelle können Störeinflüsse unterdrückt werden.

Eine besonders kompakte und somit platzsparende Vorrichtung wird dadurch erreicht, dass die Plasmadüse zwischen den zwei Elektroden angeordnet ist. Durch diese Anordnung wird die Rekombination der durch die Ionisation des Gases freigesetzten Elektronen und Ionen verhindert, da die verschieden geladenen Teilchen sofort in entgegengesetzte Richtungen zu der entsprechend entgegengesetzt geladenen Elektrode abfließen. Der Gaseinlass ist dabei bevorzugt so angeordnet, dass das Gasgemisch aus einer Richtung in den Plasmastrahl geleitet wird, die nicht parallel, vorzugsweise im Wesentlichen senkrecht zu der durch die Richtung des Plasmastrahls und die Richtung einer Verbindungslinie zwischen den beiden Elektroden aufgespannten Ebene liegt.

In einer weiteren bevorzugten Ausführungsform der Vorrichtung ist die Plasmadüse im Bereich der Kathode angeordnet. Unter der Kathode wird dabei die positiv geladene Elektrode verstanden. Auf diese Weise werden die aufgrund ihrer geringeren beweglicheren Elektronen sofort abgesaugt, so dass die Gefahr der Rekombination mit den Ionen weiter verringert ist.

Eine weitere bevorzugte Ausführungsform der Vorrichtung wird dadurch erreicht, dass die Plasmadüse zwischen dem Gaseinlass und der Kathode angeordnet ist. Die Anode befindet sich auf der von der Plasmadüse abgewandten Seite der Kathode. Die Kathode weist bevorzugt eine Öffnung auf, durch die die Ionen in den Bereich zwischen der Kathode und der Anode gelangen können. In dieser Ausführungsform ist der Plasmastrahl somit außerhalb des Zwischenraums zwischen Kathode und Anode. Somit wird das elektrische Feld zwischen Kathode und Anode nicht durch den Plasmastrahl beeinflusst.

Eine besonders geringe Temperatur des Plasmastrahls und eine dadurch bedingte geringe Aufheizung der Umgebung des Plasmastrahls wird in einer weiteren Ausführungsform der Vorrichtung dadurch erreicht, dass die Plasmadüse mindestens zwei Elektroden und eine Spannungsquelle zur Erzeugung einer hochfrequenten Hochspannung aufweist, wobei die Spannungsquelle an die mindestens zwei Elektroden angeschlossen ist.
Das Verfahren bzw. die Vorrichtung zur Detektion von ionisierbaren Gasen wird erfindungsgemäß bei einer Abgasreinigungsanlage eingesetzt Weist eine solche Anlage Mittel zur Beseitigung von ionisierbaren Molekülen, insbesondere organischen Molekülen, vorzugsweise Kohlenwasserstoffen und eine Vorrichtung zur Detektion von ionisierbaren Gasen auf, wobei die Mittel zur Beseitigung von ionisierbaren Molekülen und der Gaseinlass der Vorrichtung im Bereich einer Abgasleitung angeordnet sind, so können die Mittel zur Beseitigung von ionisierbaren Molekülen über den Wert der mit der Vorrichtung bestimmten Konzentration von ionisierbaren Gasen beispielsweise über zusätzliche Regelmittel geregelt werden.
Durch die Vorrichtung wird die Konzentration ionisierbarer Gase in dem Abgas-Gasgemisch detektiert. Die Mittel zur

Beseitigung dieser Gase kann durch die detektierte Konzentration so geregelt werden, dass die Konzentration einen bestimmten Grenzwert unterschreitet. Somit erlaubt eine solche Abgasreinigungsanlage, die Konzentration der ionisierbaren Gase zu regeln und beispielsweise festgelegte Emissionsgrenzwerte einzuhalten. Dies ist erforderlich, da durch die Mittel zur Beseitigung der ionisierbaren Moleküle in der Regel keine vollständige Beseitigung möglich ist.

In einer bevorzugten Ausführungsform ist die erfindungsgemäße Vorrichtung strömungsaufwärts oder strömungsabwärts der Mittel zur Beseitigung von ionisierbaren Molekülen angeordnet. Bei der strömungsaufwärtigen Anordnung wird die Konzentration der ionisierbaren Gase vor deren Beseitigung ermittelt, so dass die Mittel ohne Verzögerung auf die Konzentrationen eingestellt werden können. Dies ist insbesondere vorteilhaft bei zeitlich schnell schwankenden Konzentrationen. Bei der strömungsabwärtigen Anordnung wird die Konzentration der ionisierbaren Gase nach der Beseitigung, das heißt die Endkonzentration gemessen. Dadurch ist es einfacher, die Mittel auf bestimmte Grenzwerte einzuregeln. Unter der Beseitigung der ionisierbaren Moleküle ist in der Regel keine vollständige Beseitigung zu verstehen.

Weitere Merkmale und Vorteile der vorliegenden Erfindung werden in der Beschreibung von fünf Ausführungsbeispielen näher erläutert, wobei auf die beigefügte Zeichnung Bezug genommen wird. In der Zeichnung zeigen
- Fig. 1: ein erstes Ausführungsbeispiel der erfindungsgemäßen Vorrichtung sowie ein erstes Ausführungsbeispiel des erfindungsgemäßen Verfahrens,
- Fig. 2: ein zweites Ausführungsbeispiel der erfindungsgemäßen Vorrichtung sowie ein zweites Ausführungsbeispiel des erfindungsgemäßen Verfahrens,
- Fig. 3: ein drittes Ausführungsbeispiel der erfindungsgemäßen Vorrichtung sowie ein drittes Ausführungsbeispiel des erfindungsgemäßen Verfahrens,
- Fig. 4: ein viertes Ausführungsbeispiel der erfindungsgemäßen Vorrichtung sowie ein viertes Ausführungsbeispiel des erfindungsgemäßen Verfahrens,
- Fig. 5: ein fünftes Ausführungsbeispiel der erfindungsgemäßen Vorrichtung sowie ein fünftes Ausführungsbeispiel des erfindungsgemäßen Verfahrens,
- Fig. 6: ein Ausführungsbeispiel einer Vorrichtung zur spektroskopischen Gasanalyse und
- Fig. 7: ein Diagramm eines Ausführungsbeispiels einer Abgasreinigungsanlage.

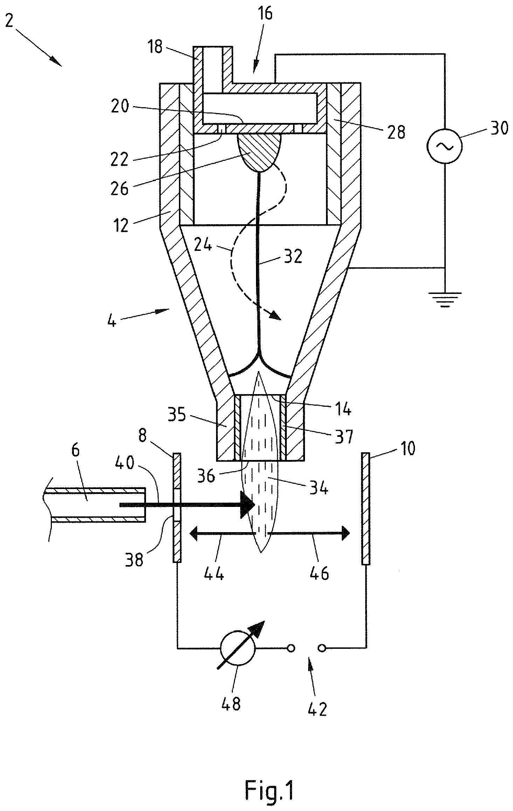

Fig. 1 zeigt ein erstes Ausführungsbeispiel der erfindungsgemäßen Vorrichtung sowie ein erstes Ausführungsbeispiel des erfindungsgemäßen Verfahrens. Die

Vorrichtung 2 weist eine Plasmadüse 4, einen Gaseinlass 6, eine erste Elektrode 8 und eine zweite Elektrode 10 auf. Die erste Elektrode 8 ist zwischen dem Gaseinlass 6 und der zweiten Elektrode 10 angeordnet. Die Plasmadüse 4 ist zwischen der ersten Elektrode 8 und der zweiten Elektrode 10 angeordnet.

Die Plasmadüse 4 weist ein Düsenrohr 12 aus Metall auf, das sich konisch zu einem Düsenrohrauslass 14 verjüngt. Am dem Düsenrohrauslass 14 entgegengesetzten Ende weist das Düsenrohr 12 eine Dralleinrichtung 16 mit einem Einlass 18 für ein Arbeitsgas auf, beispielsweise für Stickstoff oder Luft. Eine Zwischenwand 20 der Dralleinrichtung 16 weist einen Kranz von schräg in Umfangsrichtung angestellten Bohrungen 22 auf, durch die das Arbeitsgas verdrallt wird. Der stromabwärtige, konisch verjüngte Teil des Düsenrohres wird deshalb von dem Arbeitsgas in der Form eines Wirbels 24 durchströmt, dessen Kern auf der Längsachse des Düsenrohres verläuft.

An der Unterseite der Zwischenwand 20 ist mittig eine Elektrode 26 angeordnet, die koaxial in Richtung des verjüngten Abschnittes in das Düsenrohr hineinragt. Die Elektrode 26 ist elektrisch mit der Zwischenwand 20 und den übrigen Teilen der Dralleinrichtung 16 verbunden. Die Dralleinrichtung 16 ist durch ein Keramikrohr 28 elektrisch gegen das Düsenrohr 12 isoliert. Über die Dralleinrichtung 16 wird an die Elektrode 26 eine hochfrequente Hochspannung angelegt, die von einem Transformator 30 erzeugt wird. Der Einlass 18 ist über einen nicht gezeigten Schlauch mit einer unter Druck stehenden Arbeitgasquelle mit variablem Durchsatz verbunden. Das Düsenrohr 12 ist geerdet.

Durch die angelegte Spannung wird eine Hochfrequenzentladung in der Form eines Lichtbogens 32 zwischen der Elektrode 26 und dem Düsenrohr 12 erzeugt. Das Düsenrohr 12 stellt somit die zweite Elektrode dar. Unter einer Bogenentladung wird hier ein solcher Lichtbogen verstanden. Der Begriff "Lichtbogen" bzw. der in dieser Schrift synonym verwendete Begriff "Bogenentladung" wird hier als phänomenologische Beschreibung der Entladung verwendet, da die Entladung in Form eines Lichtbogens auftritt.

Aufgrund der drallförmigen Strömung des Arbeitsgases wird dieser Lichtbogen jedoch im Wirbelkern auf der Achse des Düsenrohres 12 kanalisiert, so dass er sich erst im Bereich des Düsenrohrauslasses 14 zur Wand des Düsenrohres 12 verzweigt. Das Arbeitsgas, das im Bereich des Wirbelkerns und damit in unmittelbarer Nähe des Lichtbogens 32 mit hoher Strömungsgeschwindigkeit rotiert, kommt mit dem Lichtbogen in innige Berührung und wird dadurch zum Teil in den Plasmazustand überführt, so dass ein atmosphärischer Plasmastrahl 34 durch den Düsenrohrauslass 14, durch den Auslassbereich 35 und durch die Auslassöffnung 36 aus der Plasmadüse 4 austritt. Der Auslassbereich 35 kann optional ein isolierendes Keramikrohr 37 aufweisen.

Alternativ kann anstelle des Transformators 30 auch eine gesteuerte Gleichspannungsquelle vorgesehen sein. Diese weist vorzugsweise eine Stromregelung bzw. eine Strombegrenzung auf. Durch die angelegte Gleichspannung wird dann eine kurzzeitige Entladung in Form eines Funkens bzw. eine dauerhafte Entladung in Form eines Lichtbogens zwischen der Elektrode 26 und dem Düsenrohr 12 erzeugt.

Die erste Elektrode 8 weist eine Öffnung 38 auf. Durch diese Öffnung 38 gelangt ein durch den Gaseinlass 6 einströmendes Gasgemisch 40 in den Bereich zwischen der ersten Elektrode 8 und der zweiten Elektrode 10. Das Gasgemisch 40 tritt dort mit dem Plasmastrahl 34 in Wechselwirkung. Dadurch werden die Moleküle des ionisierbaren Gases in Ionen und Elektronen aufgespalten. Bei Kohlenwasserstoffen entstehen zum Beispiel CHO⁺-Ionen.

Zwischen der ersten Elektrode 8 und der zweiten Elektrode 10 ist mit einer Spannungsquelle 42 eine Spannung von beispielsweise 100 V angelegt. Bei der Spannungsquelle 42 kann es sich um eine Gleichspannungsquelle handeln, wobei die erste Elektrode 8 an den Plus-Pol angeschlossen ist. Die erste Elektrode 8 stellt damit die Kathode dar. Die an den Minus-Pol angeschlossene zweite Elektrode 10 stellt entsprechende die Anode dar. Alternativ kann es sich bei der Spannungsquelle 42 auch um eine Wechselspannungsquelle handeln. Durch das elektrische Feld zwischen der ersten Elektrode 8 und der zweiten Elektrode 10 werden dann die freien Elektronen 44 von der ersten Elektrode 8 und die positiv geladenen Ionen 46 von der zweiten Elektrode 10 angezogen. Der dadurch fließende elektrische Strom wird über ein mit der Spannungsquelle 42 in Reihe geschaltetes Strommessgerät 48 gemessen. Der gemessene Stromwert ist ein Maß für die Konzentration des ionisierbaren Gases im Gasgemisch 40.

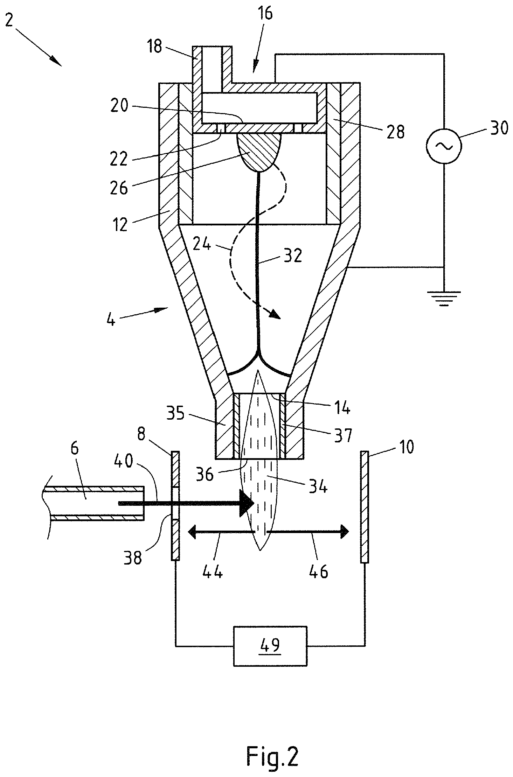

Fig. 2 zeigt ein zweites Ausführungsbeispiel der erfindungsgemäßen Vorrichtung und ein zweites Ausführungsbeispiel des erfindungsgemäßen Verfahrens. Dieses Ausführungsbeispiel unterscheidet sich von dem vorherigen dadurch, dass zwischen der ersten Elektrode 8 und der zweiten Elektrode 10 ein Modul 49 angeschlossen ist. Das Modul 49 stellt dabei zusammen mit den Elektroden 8, 10 einen elektrischen Resonanzkreis dar. Beispielsweise weist das Modul 49 einen Widerstand und eine Induktivität auf. Die Elektrodenanordnung der Elektroden 8, 10 stellt im Wesentlichen eine Kapazität dar. Im Modul 49 kann auch eine zusätzliche Kapazität vorgesehen sein. Das Modul 49 kann weiterhin eine Spannungsquelle aufweisen, durch die der Resonanzkreis angeregt werden kann. Weiterhin sind im Modul Mittel vorgesehen, um die Resonanzfrequenz des Resonanzkreises zu bestimmen. Durch die Ionen 46 bzw. durch die Elektronen 44 ändert sich die Kapazität der Elektrodenanordnung und somit die Resonanzfrequenz des Resonanzkreises. Diese stellt somit ein Maß für die Ionen 46 bzw. die Elektronen 44 dar.

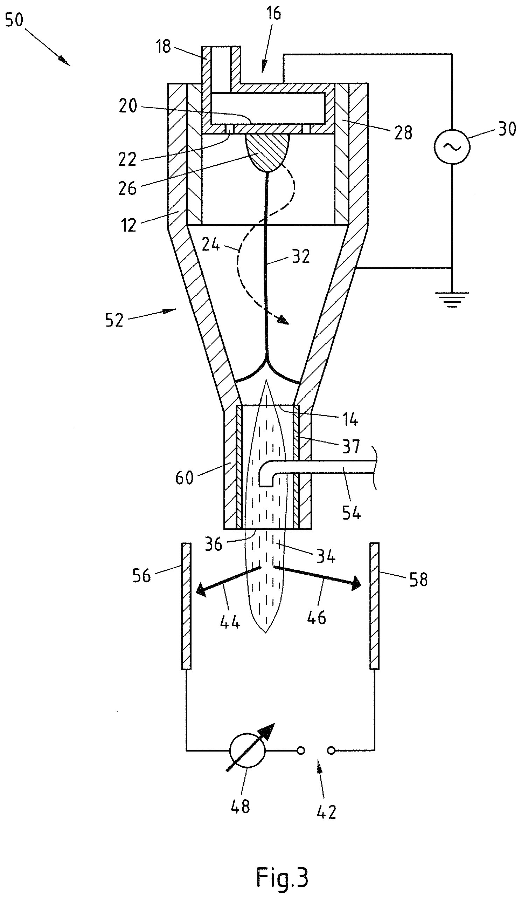

Fig. 3 zeigt ein drittes Ausführungsbeispiel der erfindungsgemäßen Vorrichtung und ein drittes Ausführungsbeispiel des erfindungsgemäßen Verfahrens. Die Vorrichtung 50 weist eine Plasmadüse 52, einen Gaseinlass 54, eine erste Elektrode 56 und eine zweite Elektrode 58 auf. Die Plasmadüse 52 unterscheidet sich von der in Fig. 1 gezeigten Plasmadüse 4 darin, dass die Plasmadüse 52 einen verlängerten Auslassbereich 60 aufweist. Der Gaseinlass 54 ist im Auslassbereich 60 angeordnet, so dass das durch den Gaseinlass 54 einströmende Gasgemisch noch im Auslassbereich 60 in den Plasmastrahl 34 gelangt.

Das in dem Gasgemisch enthaltene ionisierbare Gas wird in dem Plasmastrahl 34 ionisiert. Sobald das ionisierte Gas in den Bereich zwischen der ersten Elektrode 56 und der zweiten Elektrode 58 gelangt ist, bewegen sich die beim Ionisieren entstandenen freien Elektronen 44 zur ersten Elektrode 56 und die positiv geladenen Ionen 46 zur zweiten Elektrode 58. Durch das Einleiten des Gasgemisches in den Auslassbereich 60 wird verhindert, dass ein unionisierter Teil des ionisierbaren Gases in den Bereich zwischen die zwei Elektroden gelangt und dort beispielsweise die freien Elektronen 44 oder die Ionen 46 beeinflusst.

Natürlich kann zwischen den Elektroden 56, 58 alternativ zu der gezeigten Spannungsquelle 42 und dem Strommessgerät 38 auch ein Modul wie im zweiten Ausführungsbeispiel angeordnet werden.

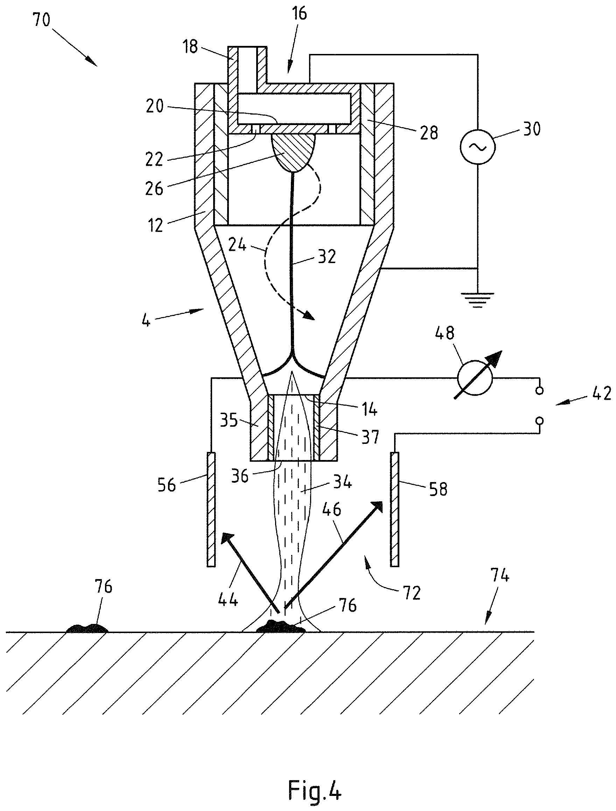

Fig. 4 zeigt ein viertes Ausführungsbeispiel der erfindungsgemäßen Vorrichtung und ein viertes Ausführungsbeispiel des erfindungsgemäßen Verfahrens. Die Vorrichtung 70 weist eine wie in Fig. 1 dargestellte Plasmadüse 4, eine erste Elektrode 56 und eine zweite Elektrode 58 auf. Der Gaseinlass ist bei der Vorrichtung 70 als untere Öffnung 72 zwischen den zwei Elektroden ausgebildet. Die Vorrichtung 70 ist über einer Oberfläche 74 positioniert, welche Verschmutzungen 76 aufweist. Bei der Verschmutzung kann es sich beispielsweise um organische Verschmutzungen, insbesondere um Kohlenwasserstoffe handeln.

Der aus der Plasmadüse 4 austretende Plasmastrahl 34 verläuft durch den Bereich zwischen den zwei Elektroden und trifft auf die Oberfläche 74. Durch die im Plasmastrahl 34 zur Verfügung gestellte Energie wird die Verschmutzung 76 nach und nach von der Oberfläche 74 abgelöst. Die von der Oberfläche 74 abgelösten, ionisierbaren Moleküle vermischen dann mit dem Umgebungsgas zu einem Gasgemisch, welches durch die untere Öffnung 72 in die Vorrichtung 70 gelangt. Die Moleküle werden in dem Plasmastrahl 34 ionisiert. Die dadurch entstehenden freien Elektronen 44 bewegen sich zur ersten Elektrode 56 und die positiv geladenen Ionen 46 zur zweiten Elektrode 58.

Die Oberfläche 74 kann beispielsweise so lange mit dem Plasmastrahl 34 beaufschlagt werden, bis der mit dem Strommessgerät 48 gemessene elektrische Strom einen festgelegten Wert unterschreitet. Dadurch wird erreicht, dass die Beaufschlagung der Oberfläche 74 erst endet, wenn ein gewisser Teil der Verschmutzung 76 entfernt wurde.

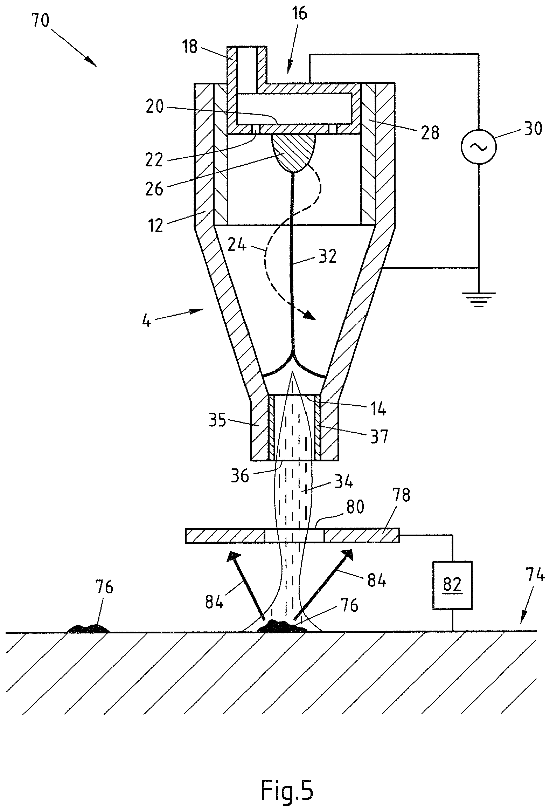

Fig. 5 zeigt ein fünftes Ausführungsbeispiel der erfindungsgemäßen Vorrichtung und ein fünftes Ausführungsbeispiel des erfindungsgemäßen Verfahrens. Dieses Ausführungsbeispiel unterscheidet sich vom vierten Ausführungsbeispiel dadurch, dass eine Elektrode 78 mit einer Öffnung 80 vorgesehen ist, durch die der Plasmastrahl 34 hindurch tritt. Weiterhin ist ein Modul 82 mit der Elektrode 78 und mit der Oberfläche 74 elektrisch verbunden. Die Oberfläche 74 stellt somit die zweite Elektrode dar. Die Oberfläche ist daher vorzugsweise elektrisch leitend. Das Modul 82 kann beispielsweise eine Spannungsquelle und ein Strommessgerät aufweisen. Alternativ kann das Modul 82 mit der Elektrodenanordnung der Elektrode 78 und der Oberfläche 74 einen Resonanzkreis bilden. Liegt an der Elektrode 78 eine relativ zur Oberfläche 74 negative Spannung an, so werden die bei der Ionisierung durch den Plasmastrahl 34 entstehenden Ionen 84 auf die Elektrode 74 zu bewegt. Die Elektronen (nicht gezeigt) fließen über die Oberfläche 74 ab. Bei umgekehrter Polarität verhält es sich entsprechend umgekehrt.

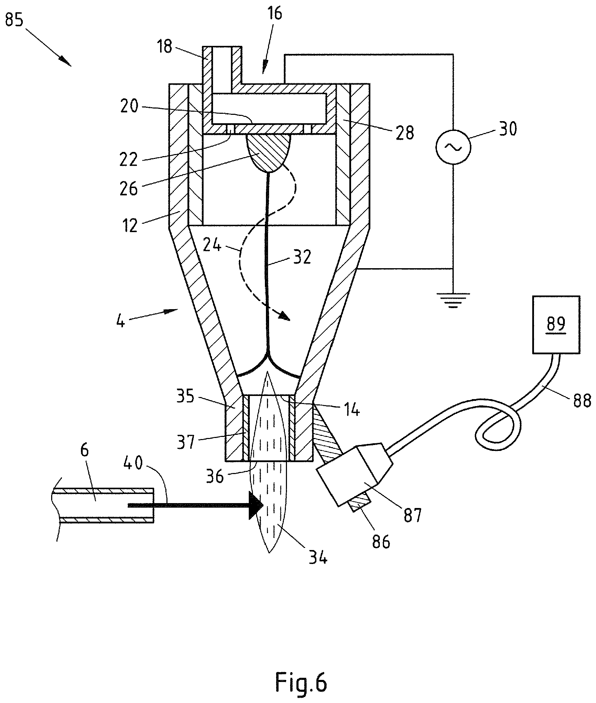

Fig. 6 zeigt ein Ausführungsbeispiel einer Vorrichtung zur spektroskopischen Gasanalyse. Die Plasmadüse 85 unterscheidet sich von der in Fig. 1 gezeigten Plasmadüse 2 dadurch, dass zusätzlich an einer Halterung 86 befestigte optische Erfassungsmittel 87 vorgesehen sind, welches auf den Bereich ausgerichtet ist, in dem das Gasgemisch 40 mit dem Plasmastrahl 34 in Wechselwirkung gebracht wird. Das von den angeregten Stoffen aus diesem Bereich emittierte Licht wird zumindest teilweise von den Erfassungsmitteln 87 erfasst und über einen Lichtleiter 88 zu einem Spektrometer 89 geführt. Im Spektrometer 89 wird dann die Intensität in mindestens einem Spektralbereich gemessen und mit diesem Messwert die Konzentration mindestens eines Stoffes im Gasgemisch 40 ermittelt. Die Erfassungsmittel 87 können zur besseren Erfassung des Lichtes optional eine Sammeloptik aufweisen. Das Spektrometer 89 ist bevorzugt als Spektrometer für die Optische Emissionsspektroskopie ausgebildet. Als Erfassungsmittel 87 können auch ein energiedispersiver Detektor verwendet werden, so dass auf ein separates Spektrometer verzichtet werden kann. Die Erfassungsmittel 87 können weiterhin separat von der Plasmadüse 85 angeordnet werden.

Natürlich ist es möglich, die Merkmale der vorherigen Ausführungsbeispiele miteinander zu kombinieren. So kann das durch den Plasmastrahl 34 ionisierte bzw. angeregte Gasgemisch 40 beispielsweise in vorteilhafter Weise gleichzeitig über vorgesehene Elektroden, wie in den Figs. 1 bis 5 dargestellt, und spektroskopisch, wie in Fig. 6 dargestellt, analysiert werden.

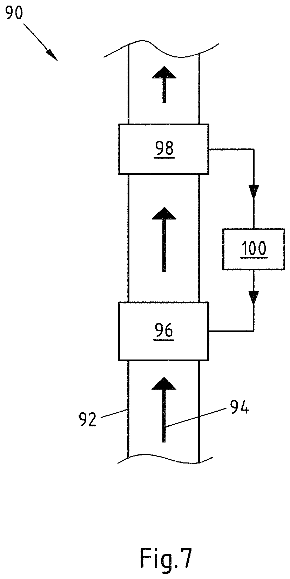

Fig. 7 zeigt ein Diagramm eines Ausführungsbeispiels einer Abgasreinigungsanlage. Die Abgasreinigungsanlage 90 weist eine Abgasführung 92 auf, in der ein Abgas 94 geführt wird. Bei dem Abgas 94 kann es sich beispielsweise um ein Abgasgemisch aus einem Verbrennungsprozess handeln, welches ein ionisierbares Gas enthält. Das Abgas 94 gelangt zunächst zu den Mitteln 96 zur Beseitigung von ionisierbaren Molekülen. Bei den Mitteln 96 kann es sich beispielsweise um einen regelbaren Katalysator handeln. Das Abgas 94 gelangt dann weiter zu einer erfindungsgemäßen Vorrichtung 98 zur Detektion von ionisierbaren Gasen, in der die Konzentration des ionisierbaren Gases im Abgas 94 detektiert wird.

Die detektierte Konzentration des ionisierbaren Gases wird in eine Regeleinheit 100 gespeist, welche ein Regelsignal zur Regelung der Mittel 96 erzeugt. Die Regeleinheit ist dabei so eingerichtet, dass der Beseitigungsgrad der Mittel 96 bei einer zu hohen durch die Vorrichtung 98 detektierten Konzentration des ionisierbaren Gases erhöht wird. Dadurch wird gewährleistet, dass die Konzentration des ionisierbaren Gases im Abgas 94 nach dem Durchlaufen der Abgasreinigungsanlage 90 unterhalb eines vorgegebenen Grenzwertes liegt.

Alternativ ist es auch möglich, dass die Vorrichtung 98 stromaufwärts der Mittel 96 angeordnet wird.

## Patentansprüche

1. Verfahren zur Detektion von ionisierbaren Gasen, insbesondere organischen Molekülen, vorzugsweise Kohlenwasserstoffen, einer Abgasreinigungsanlage,
- bei dem ein Gasgemisch (40) aus einem Abgasfluss abgeleitet wird,
- bei dem ein aus einer Plasmadüse austretender atmosphärischer Plasmastrahl (34) erzeugt wird,
- bei dem das ein ionisierbares Gas enthaltende Gasgemisch (40) mit dem Plasmastrahl (34) in Wechselwirkung gebracht wird, so dass das ionisierbare Gas durch den Plasmastrahl zumindest teilweise ionisiert wird unter Entstehung beweglicher Ladungsträger, und bei dem ein durch die beweglichen Ladungsträger bewirkter elektrischer Strom als Maß für die Konzentration des ionisierbaren Gases in dem Gasgemisch (40) gemessen wird.

2. Verfahren nach Anspruch 1,
**dadurch gekennzeichnet,**
**dass** der elektrische Strom zwischen zwei Elektroden (8, 10) gemessen wird, wobei zwischen den Elektroden eine Spannung angelegt wird.

3. Verfahren nach Anspruch 2,
**dadurch gekennzeichnet,**
**dass** zwischen den Elektroden (8, 10) eine Spannung zwischen 50 V und 350 V, insbesondere zwischen 50 V und 250 V, vorzugsweise zwischen 75 V und 150 V angelegt wird.

4. Verfahren nach Anspruch 2 oder 3,
**dadurch gekennzeichnet, dass**
zwischen den Elektroden (8, 10) eine Gleichspannung oder eine Wechselspannung angelegt ist.

5. Verfahren nach einem der Ansprüche 1 bis 4,
**dadurch gekennzeichnet,**
**dass** die Abgasreinigungsanlage Mittel zur Beseitigung von ionisierbaren Molekülen, insbesondere organischen Molekülen, vorzugsweise Kohlenwasserstoffen, aufweist.

6. Verfahren nach Anspruch 5,
**dadurch gekennzeichnet,**
**dass** die Mittel zur Beseitigung von ionisierbaren Molekülen über den ermittelten Wert der Konzentration des ionisierbaren Gases geregelt werden.

7. Abgasreinigungsanlage,
mit einer Vorrichtung zur Detektion von ionisierbaren Gasen, insbesondere organischen Molekülen, vorzugsweise Kohlenwasserstoffen,
**dadurch gekennzeichnet,**
- dass die Vorrichtung
einen Gaseinlass (6),
Mittel zur Ionisation eines ionisierbaren Gases, eine Spannungsquelle (42),
zwei Elektroden (8, 10) und
Mittel (48) zur Bestimmung einer Stromstärke, aufweist,
- dass als Mittel zur Ionisation eines ionisierbaren Gases eine Plasmadüse (4) zur Erzeugung eines atmosphärischen Plasmastrahls (34) vorgesehen ist,
- dass die zwei Elektroden (8, 10) an die Spannungsquelle (42) angeschlossen sind und
- dass die Mittel (48) zur Bestimmung einer Stromstärke so an die Elektroden (8, 10) angeschlossen sind, dass die Stärke des zwischen den Elektroden (8, 10) fließenden Stroms messbar ist, der durch bei der Ionisation des ionisierbaren Gases entstandene bewegliche Ladungsträger bewirkt wird.

8. Abgasreinigungsanlage nach Anspruch 7,
**dadurch gekennzeichnet,**
**dass** mit der Spannungsquelle (42) eine Spannung zwischen 50 V und 350 V, insbesondere zwischen 50 V und 250 V, vorzugsweise zwischen 75 V und 150 V erzeugbar ist.

9. Abgasreinigungsanlage nach Anspruch 7 oder 8,
**dadurch gekennzeichnet,**
**dass** als Spannungsquelle (42) eine Gleichspannungsquelle vorgesehen ist.

10. Abgasreinigungsanlage nach einem der Ansprüche 7 bis 9,
**dadurch gekennzeichnet,**
**dass** die Plasmadüse (4) zwischen den zwei Elektroden (8, 10) angeordnet ist.

11. Abgasreinigungsanlage nach Anspruch 9,
**dadurch gekennzeichnet,**
**dass** die Plasmadüse im Bereich der Kathode angeordnet ist.

12. Abgasreinigungsanlage nach Anspruch 9,
**dadurch gekennzeichnet,**
**dass** die Plasmadüse (4) zwischen dem Gaseinlass und der Kathode (8) angeordnet ist.

13. Abgasreinigungsanlage nach einem der Ansprüche 7 bis 12,
**dadurch gekennzeichnet,**
**dass** die Plasmadüse (4) mindestens zwei Elektroden (26, 12) und eine Spannungsquelle (30) zur Erzeugung einer hochfrequenten Hochspannung aufweist, wobei die Spannungsquelle an die mindestens zwei Elektroden (26, 12) angeschlossen ist.

14. Abgasreinigungsanlage nach einem der Ansprüche 7 bis 13,
**dadurch gekennzeichnet, dass**
Mittel zur Beseitigung von ionisierbaren Molekülen, insbesondere organischen Molekülen, vorzugsweise Kohlenwasserstoffen, vorgesehen sind.

15. Abgasreinigungsanlage nach Anspruch 14,
**dadurch gekennzeichnet, dass**
Mittel zur Regelung der Mittel zur Beseitigung von ionisierbaren Molekülen über den ermittelten Wert der Konzentration des ionisierbaren Gases vorgesehen sind.

## Claims

1. Method for detecting ionisable gases, in particular organic molecules, preferably hydrocarbons, of an exhaust gas purification system,
- wherein a gas mixture (40) is derived from an exhaust gas stream,
- wherein an atmospheric plasma jet (34) exiting from a plasma nozzle is generated,
- wherein a gas mixture (40) containing the ionisable gas is made to interact with the plasma jet (34), such that the ionisable gas is at least partially ionised by the plasma jet resulting in movable charge carriers, and wherein an electrical current generated by the movable charge carriers is measured so as to be used as a measure for the concentration of the ionisable gas in the gas mixture (40).

2. Method according to Claim 1, **characterised in that** the electrical current is measured between two electrodes (8, 10), with a voltage being applied between the electrodes.

3. Method according to Claim 2, **characterised in that** a voltage between 50 V and 350 V, in particular between 50 V and 250 V, preferably between 75 V and 150 V is applied between the electrodes (8, 10).

4. Method according to Claim 2 or 3, **characterised in that** a DC voltage or an AC voltage is applied between the electrodes (8, 10).

5. Method according to any one of Claims 1 to 4, **characterised in that** the exhaust gas purification system comprises means for removing ionisable molecules, in particular organic molecules, preferably hydrocarbons.

6. Method according to claim 5,
**characterized in that** the means for removing ionisable molecules are controlled via the determined value of the concentration of ionisable gases.

7. Exhaust gas purification system, with an apparatus for detecting ionisable gases, in particular organic molecules, preferably hydrocarbons,
**characterized in**
- **that** the apparatus comprises a gas inlet (6), means for ionising an ionisable gas, a voltage source (42), two electrodes (8, 10) and means (48) for determining a current strength,
- **that** a plasma nozzle (4) for generating an atmospheric plasma jet (34) is provided as means for ionising an ionisable gas,
- **that** the two electrodes (8, 10) are connected to the voltage source (42), and
- **that** the means (48) for determining a current strength are connected to the electrodes (8, 10) such that the strength of the current flowing between the electrodes (8, 10) can be measured, which current is generated by movable charge carriers resulting from the ionising of the ionisable gas.

8. Exhaust gas purification system according to Claim 7, **characterised in that** a voltage between 50 V and 350 V, in particular between 50 V and 250 V, preferably between 75 V and 150 V can be generated using the voltage source (42).

9. Exhaust gas purification system according to Claim 7 or 8, **characterised in that** a DC voltage source is provided as the voltage source (42).

10. Exhaust gas purification system according to any one of Claims 7 to 9, **characterised in that** the plasma nozzle (4) is arranged between the two electrodes (8, 10).

11. Exhaust gas purification system according to Claim 9, **characterised in that** the plasma nozzle is arranged in the area of the cathode.

12. Exhaust gas purification system according to Claim 9, **characterised in that** the plasma nozzle (4) is arranged between the gas inlet and the cathode (8).

13. Exhaust gas purification system according to any one of Claims 7 to 12, **characterised in that** the plasma nozzle (4) has at least two electrodes (26, 12) and a voltage source (30) for generating a high-frequency high voltage, said voltage source being connected to the at least two electrodes (26, 12).

14. Exhaust gas purification system according to any one of Claims 7 to 13, **characterized in that** means for removing ionisable molecules, in particular organic molecules, preferably hydrocarbons, are provided.

15. Exhaust gas purification system according to Claim 14, **characterized in that** means for controlling the means for removing ionisable molecules via the determined value of the concentration of ionisable gases are provided.

## Revendications

1. Procédé de détection de gaz ionisables, notamment de molécules organiques, de préférence d'hydrocarbures, d'une installation d'épuration de gaz d'échappement,
- selon lequel un mélange gazeux (40) est dérivé d'un gaz d'échappement,
- selon lequel un jet de plasma (34) atmosphérique sortant d'une buse à plasma est produit,
- selon lequel le mélange gazeux (40) contenant un gaz ionisable est mis en interaction avec le jet de plasma (34), de sorte que le gaz ionisable est au moins partiellement ionisé par le jet de plasma donnant naissance à des porteurs de charge mobiles, et selon lequel un courant électrique produit par les porteurs de charge mobiles est mesuré en tant que grandeur électrique indiquant la concentration du gaz ionisable dans le mélange gazeux (40).

2. Procédé selon la revendication 1, **caractérisé en ce que** le courant électrique entre deux électrodes (8, 10) est mesuré, auquel cas une tension est appliquée entre les électrodes.

3. Procédé selon la revendication 2, **caractérisé en ce qu'**une tension entre 50 V et 350 V, notamment entre 50 V et 250 V, de préférence entre 75 V et 150 V est appliquée entre les électrodes (8, 10)

4. Procédé selon la revendication 2 ou 3, **caractérisé en ce qu'**une tension continue ou une tension alternative est appliquée entre les électrodes (8, 10).

5. Procédé selon une des revendications de 1 à 4, **caractérisé en ce que** l'installation d'épuration de gaz d'échappement présente des moyens permettant l'élimination de molécules ionisables, notamment de molécules organiques, de préférence d'hydrocarbures.

6. Procédé selon la revendication 5, **caractérisé en ce que** les moyens permettant l'élimination des molécules ionisables sont réglés par l'intermédiaire de la valeur déterminée de la concentration du gaz ionisable.

7. Installation d'épuration de gaz d'échappement dotée d'un dispositif de détection de gaz ionisables, notamment de molécules organiques, de préférence d'hydrocarbures,
**caractérisé en ce que**
- le dispositif présente une entrée de gaz (6), des moyens d'ionisation d'un gaz ionisable, une source de tension (42), deux électrodes (8, 10) et des moyens (48) de détermination de l'intensité d'un courant,
- une buse à plasma (4) est prévue comme moyen d'ionisation d'un gaz ionisable pour la production d'un jet de plasma atmosphérique (34),
- les deux électrodes (8, 10) sont raccordés à la source de tension (42) et
- des moyens (48) de détermination d'une intensité de courant sont raccordés aux électrodes (8, 10) de façon que l'intensité du courant circulant entre les électrodes (8, 10) soit mesurable, le courant étant produit par les porteurs de charge mobiles formé par l'ionisation d'un gaz ionisable.

8. Installation d'épuration de gaz d'échappement selon la revendication 7, **caractérisée en ce que** l'on est susceptible de pouvoir produire avec la source de tension (42) une tension entre 50 V et 350 V, notamment entre 50 V et 250 V, de préférence entre 75 V et 150 V.

9. Installation d'épuration de gaz d'échappement selon la revendication 7 ou 8, **caractérisée en ce qu'**une source de tension continue est prévue en tant que source de tension (42).

10. Installation d'épuration de gaz d'échappement selon une des revendications de 7 à 9, **caractérisée en ce que** la buse à plasma est agencée entre les deux électrodes (8, 10).

11. Installation d'épuration de gaz d'échappement selon la revendication 9, **caractérisée en ce que** la buse à plasma est agencée dans le secteur de la cathode.

12. Installation d'épuration de gaz d'échappement selon la revendication 9, **caractérisée en ce que** la buse à plasma (4) est agencée entre l'entrée de gaz et la cathode (8).

13. Installation d'épuration de gaz d'échappement selon une des revendications de 7 à 12, **caractérisée en ce que** la buse à plasma (4) présente au moins deux électrodes (26, 12) et une source de courant (30) pour la production d'une haute tension à haute fréquence, auquel cas la source de tension est raccordée à au moins les deux électrodes (26, 12).

14. Installation d'épuration de gaz d'échappement selon une des revendications de 7 à 13 **caractérisée en ce que** l'on prévoit des moyens permettant l'élimination des molécules ionisables, notamment de molécules organiques, de préférence d'hydrocarbures.

15. Installation d'épuration de gaz d'échappement selon la revendication 14, **caractérisée en ce que** l'on prévoit des moyens permettant le réglage des moyens permettant l'élimination de molécules ionisables per l'intermédiaire de la valeur déterminée de la concentration du gaz ionisable.
